## Europäisches Patentamt
## European Patent Office
## Office européen des brevets

(19)

(11) Veröffentlichungsnummer: **0 054 728**
**B1**

(12) # EUROPÄISCHE PATENTSCHRIFT

(45) Veröffentlichungstag der Patentschrift:
**10.04.85**

(51) Int. Cl.⁴: **A 61 M 25/00**

(21) Anmeldenummer: **81109367.3**

(22) Anmeldetag: **30.10.81**

(54) **Katheterisierungsvorrichtung.**

(30) Priorität: **23.12.80 DE 3048774**

(43) Veröffentlichungstag der Anmeldung:
**30.06.82 Patentblatt 82/26**

(45) Bekanntmachung des Hinweises auf die Patenterteilung:
**10.04.85 Patentblatt 85/15**

(84) Benannte Vertragsstaaten:
**AT BE CH DE FR GB IT LI LU NL SE**

(56) Entgegenhaltungen:
**DE - A - 1 491 624**
**DE - A - 1 957 647**
**DE - A - 2 305 640**
**DE - A - 2 615 702**
**DE - A - 2 645 520**
**GB - A - 1 475 409**
**US - A - 3 585 996**

(73) Patentinhaber: **INTERMEDICAT GMBH,**
**Gerliswilstrasse 43, CH-6020 Emmenbrücke (CH)**

(72) Erfinder: **Schacht, Bodo, Weihersgrund 1,**
**D-3509 Malsfeld (DE)**

(74) Vertreter: **von Kreisler, Alek et al, Patentanwälte Von**
**Kreisler-Schönwald-Fues-Keller Selting-Werner**
**Deichmannhaus am Hauptbahnhof, D-5000 Köln 1 (DE)**

## Beschreibung

Die Erfindung betrifft eine Katheterisierungsvorrichtung, mit einem Katheter, der an seinem rückwärtigen Ende einen Katheteransatz aufweist, mit einer an ihrem rückwärtigen Ende mit einem eine Kupplungsvorrichtung aufweisenden Hülsenansatz versehenen Hülse zum Einführen des Katheters, mit einer durch die Hülse hindurchsteckbaren Kanüle, die an ihrem vorderen Ende mit einer Schneidkante und an ihrem rückwärtigen Ende mit einem Handgriff versehen ist, und mit einem von dem Katheter zu durchdringenden, bis zum Rand geschlitzten und seitlich von dem Katheter abnehmbaren Dichtungselement.

Eine bekannte Katheterisierungsvorrichtung dieser Art (DE-A-2 305 640) weist eine Hülse auf, durch die zum Zwecke des Punktierens eine Kanüle hindurchgesteckt wird. Nach erfolgter Punktion verbleibt die Hülse zunächst im Körper, während die Kanüle zurückgezogen wird. Anschliessend wird in das rückwärtige Ende der Hülse ein teilbares Zwischenstück eingesteckt, das seinerseits mit einer Halterung für einen die wesentliche Länge des Katheters enthaltenden Schutzbeutel verbunden ist. Der Katheter wird aus dem Schutzbeutel heraus durch das Zwischenstück und die Hülse hindurch in die punktierte Vene eingeschoben. Danach wird das Zwischenstück entlang seiner Teilungslinien auseinandergenommen und von dem Katheter entfernt, nachdem zuvor der Schutzbeutel vom rückwärtigen Ende des Katheters abgezogen wurde. Das rückwärtige Ende des Katheters wird mit einer Fixierplatte auf der Haut des Patienten mit Hilfe von Klebepflaster festgelegt und der Katheteransatz wird mit einer Transfusions- und Infusionsleitung verbunden. Das geteilte Zwischenstück und der Schutzbeutel sind erforderlich, um eine Kontaminierung des Katheters während des Einführens zu vermeiden. Da sich an dem rückwärtigen Ende des Katheters der verdickte Katheteransatz befindet, kann zwar der Schutzbeutel über den Katheter nach hinten abgezogen werden, nicht aber das Zwischenstück. Aus diesem Grunde ist das Zwischenstück teilbar gemacht, d.h. es besteht aus zwei gegeneinandergesetzten Hälften, die radial auseinandergezogen werden können. Das Zwischenstück enthält in einem Hohlraum ein rohrförmiges elastisches Formteil, das an seinem vorderen Ende als konisch zulaufendes Dichtungselement ausgebildet ist, durch welches der Katheter hindurchgeschoben werden kann. Das Formteil ist in Längsrichtung mit einem durchgehenden Schlitz versehen, damit es nach dem Einführen des Katheters und nach dem Entfernen des Zwischenstücks vom Katheter getrennt werden kann. Da das Zwischenstück erst nach dem Entfernen der Kanüle an den Hülsenansatz der Hülse angekuppelt wird, erfolgt bei der Punktion keine Abdichtung der Körperflüssigkeit, die zwischen Hülse und Kanüle nach aussen dringen kann.

Bekannt ist ferner eine Katheterisierungsvorrichtung (US-A-3 585 986), bei der der Katheter durch die Punktionskanüle hindurch in den Körper des Patienten eingeschoben wird. Am Ende der Punktionskanüle ist eine von dem Katheter zu durchdringende Dichtungsscheibe in einem Hohlraum eines Kanülenhalters angeordnet. Der Kanülenhalter muss zusammen mit der Kanüle über das rückwärtige Katheterende abgestreift werden, um entfernt zu werden.

Schliesslich ist eine Katheterisierungsvorrichtung bekannt (DE-A-2 645 520), bei der in einem Hohlraum des Hülsenansatzes einer ungeteilten Einführhülse ein Dichtungselement angeordnet ist, durch das der Katheter hindurchführbar ist und das durch einen Sprengring in seiner Position gesichert ist.

Es ist ferner bekannt, bei einer derartigen Katheterisierungsvorrichtung auch die Hülse geteilt bzw. in Längsrichtung teilbar auszuführen, so dass nach dem Einführen des Katheters die Hülse zunächst entlang des Katheters aus dem Körper herausgezogen wird und anschliessend ihre beiden Teile durch radiales Auseinanderziehen voneinander getrennt werden. Bei Anwendung dieser bekannten Katheterisierungsvorrichtung wird die Hülse nach dem Herausziehen der Punktionskanüle manuell verschlossen, um das Auslaufen von Körperflüssigkeit zu vermeiden. Das Einsetzen eines Ventiles in die Hülse zur Verhinderung des Auslaufens von Körperflüssigkeit ist konstruktiv insbesondere bei einer teilbaren Kanüle, deren Teile radial auseinanderziehbar sind, schwierig.

Der Erfindung liegt die Aufgabe zugrunde, eine Katheterisierungsvorrichtung der eingangs genannten Art so auszubilden, dass das Auslaufen von Körperflüssigkeit nach dem Entfernen der Punktionskanüle ohne manuellen Eingriff vermieden wird.

Zur Lösung dieser Aufgabe ist erfindungsgemäss vorgesehen, dass das Dichtungselement eine vor dem Einführen des Katheters von der Kanüle zu durchdringende Dichtungsscheibe ist, die an der rückwärtigen Stirnfläche des Hülsenansatzes der in Längsrichtung teilbaren Hülse angeordnet ist und von einem mit der Kupplungsvorrichtung lösbar ineinandergreifenden Muffenteil gegen die Stirnfläche gedrückt wird, welches an seinem rückwärtigen Ende einen Durchtrittskanal aufweist.

Die das Ventil bildende Dichtungsscheibe wird von dem Muffenteil, das auf dem rückwärtigen Ende der Hülse abnehmbar befestigt ist, festgehalten. Nach dem Einführen des Katheters kann das Muffenteil von dem rückwärtigen Ende der Hülse gelöst und die Dichtungsscheibe entfernt werden.

In vorteilhafter Weiterbildung der Erfindung weist der Katheter in der Nähe seines rückwärtigen Endes eine schräge Ringschulter auf, über die das Muffenteil nach hinten schiebbar, aber nicht nach vorne zurückbewegbar ist, und es ist eine am Körper zu befestigende Fixierplatte vorgesehen, die eine mit dem Muffenteil ineinandergreifende zweite Kupplungsvorrichtung aufweist.

Nach dem Einführen des Katheters wird das Muffenteil zum Festhalten der Dichtungsscheibe nicht mehr benötigt. Da es den Katheter aber umgibt und nicht über das verdickte rückwärtige Ende des Katheters hinweg abgeschoben werden kann, wird es in sinnvoller Weise zur Befestigung des rückwärtigen Katheterendes an der Fixierplatte benutzt. Die Fixierplatte ist zu diesem Zweck mit der zweiten Kupplungsvorrichtung versehen, die in der gleichen Weise mit dem Muffenteil zusammengreifen kann wie die an dem rückwärtigen Ende der Hülse vorgesehene erste Fixiervorrichtung. Auf diese Weise kann das Muffenteil zunächst als Halterung für die Dichtungsscheibe benutzt werden, und wenn sie hierzu nicht mehr benötigt wird, wird sie auf dem Katheter zurückgeschoben und als Befestigungsteil zum Festlegen des rückwärtigen Katheterendes an der Fixierplatte benutzt.

In vorteilhafter Weiterbildung der Erfindung ist vorgesehen, dass die Hülse einen vor der Dichtungsscheibe angeordneten radial geschlitzten Dichtungszylinder enthält. Der Dichtungszylinder verhindert den Blutaustritt durch die axialen Schlitze der Hülse und dient gemeinsam mit der Dichtungsscheibe als Katheterbremse.

Bei einer bevorzugten Ausführungsform der Erfindung ist der Durchtrittskanal des Muffenteils konisch ausgebildet, und ein von dem Katheter passierbares teilbares Zwischenstück, das an seinem rückwärtigen Ende mit der Halterung eines Schutzbeutels verbunden ist, ist mit einem nach vorne weisenden hohlen Konusansatz in den Durchtrittskanal des Muffenteiles eingeschoben. Im Stand der Technik ist normalerweise der Konusansatz des Zwischenstückes in das rückwärtige Ende der Hülse eingeschoben. Nach der Erfindung dient jedoch das Muffenteil zur Befestigung des Zwischenstückes und das Muffenteil ist seinerseits unter Zwischenschaltung der Dichtungsscheibe mit der Hülse verbunden.

Im folgenden wird unter Bezugnahme auf die Zeichnungen ein Ausführungsbeispiel der Erfindung näher erläutert.

Es zeigen:

Fig. 1 einen Längsschnitt durch die Hülse mit aufgesetztem Muffenteil vor dem Einschieben der Punktionskanüle,

Fig. 1a in perspektivischer Darstellung eine Ansicht des rückwärtigen Endes der Hülse mit der Kupplungsvorrichtung,

Fig. 2 einen Längsschnitt durch die Katheterisierungsvorrichtung während des Einführens des Katheters, der aus einem Schutzbeutel heraus vorgeschoben wird und

Fig. 3 eine Draufsicht auf die Fixierplatte mit dem an ihr befestigten Katheterende.

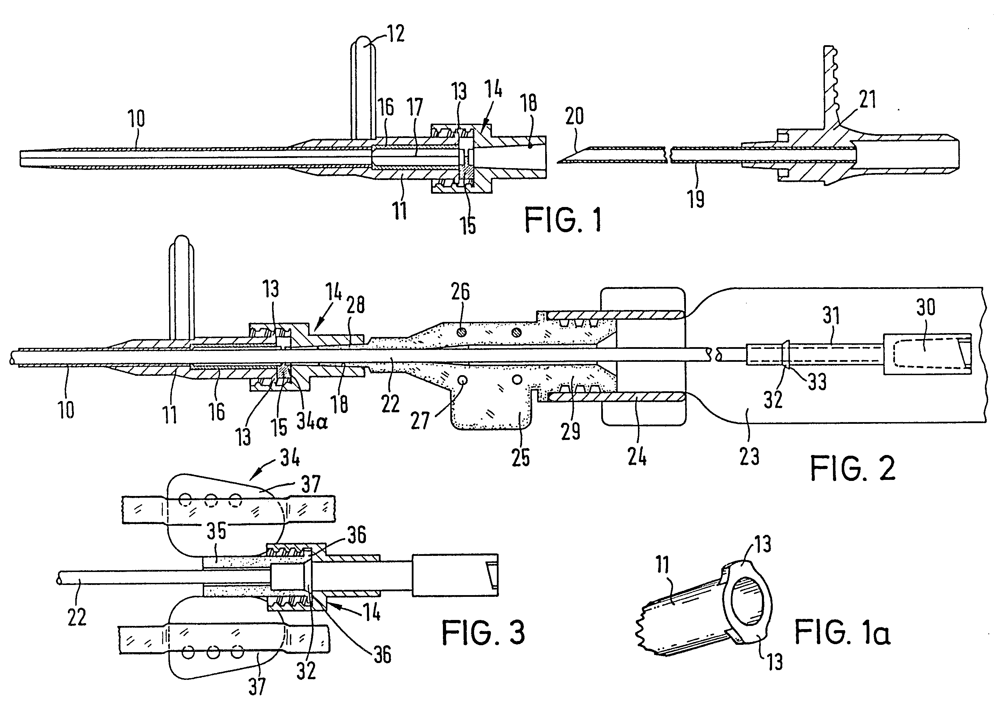

Gemäss Fig. 1 weist die teilbare Hülse 10 an ihrem rückwärtigen Ende einen teilweise gesplitteten Hülsenansatz 11 auf, an dem ein radial abstehender Handgriff 12 befestigt ist. Am rückwärtigen Ende des Hülsenansatzes 11 befinden sich zwei radial nach entgegengesetzten Richtungen abstehende Kupplungselemente 13 (Fig. 1a), die in das Innengewinde eines Muffenteiles 14 eingreifen. Zwischen der Stirnwand der Gewindeöffnung des Muffenteiles 14 und der rückwärtigen Stirnwand des Hülsenansatzes 11 ist die Dichtungsscheibe 15 eingespannt, deren Rand eine grössere Stärke aufweist als der Mittelbereich. Die Dichtungsscheibe 15 besteht aus Gummi oder Kunststoff und weist einen Radialschlitz auf, was dadurch angedeutet ist, dass ihre obere Hälfte nicht schraffiert ist. Die Dichtungsscheibe 15 besteht aus relativ weichem Gummi oder Kunststoff.

Im Innern des Hülsenansatzes 11 ist ferner ein Dichtungszylinder 16 angeordnet, der einen über seine gesamte Länge laufenden Schlitz 17 aufweist.

Das rückwärtige Ende des Muffenteiles 14 weist einen sich konisch nach aussen hin erweiternden Durchtrittskanal 18 auf, dessen vorderes Ende mit dem schwächeren Bereich der Dichtungsscheibe 15 fluchtet.

Vor dem Punktieren wird die Stahlkanüle 19, die an ihrem vorderen Ende eine abgeschrägte Schneidkante 20 und an ihrem rückwärtigen Ende einen Handgriff 21 aufweist, durch den Durchtrittskanal 18 hindurch in das Innere der Hülse 10 eingeführt, wobei der Schlitz der Dichtungsscheibe 15 geöffnet wird. In voll eingestecktem Zustand ragt die Schneidkante 20 über das vordere Ende der Hülse 10 hinaus, während der Handgriff 21 gegen das rückwärtige Ende des Muffenteiles 14 nach Art eines Anschlages stösst.

Wenn das vordere Ende der Hülse 10 in die Vene eingeführt worden ist, wird die Kanüle 19 zurückgezogen und entfernt. Dabei schliesst sich die Perforationsstelle in der Dichtungsscheibe 15 so weit wieder, dass praktisch kein Blut aus dem rückwärtigen Ende der Hülse 10 austreten kann. Innerhalb des Hülsenansatzes 11 verhindert der Dichtungszylinder 16 das Austreten von Blut durch die Teilungsschlitze.

Nun wird der Katheter 22, der sich zunächst in einem keimdicht verschlossenen, durchsichtigen, flexiblen Schutzbeutel 23 befand, aus der Halterung 24 des Schutzbeutels 23 heraus durch ein mit der Halterung 24 lösbar verbundenes teilbares Zwischenstück 25 hindurch in den Durchtrittskanal 18 eingeführt. Das vordere Ende des Katheters durchstösst die schon geschlitzte Dichtungsscheibe 15 und gelangt durch den Zylinder 16 hindurch in die Hülse 10 und über deren vorderes Ende hinaus in die Vene.

Das Zwischenstück 25 besteht aus zwei radial auseinanderziehbaren Gehäusehälften, von denen in Fig. 2 aus Gründen der Übersichtlichkeit nur eine dargestellt ist. Die Gehäusehälften weisen ineinandergreifende Zapfen 26 und Löcher 27 auf. Das vordere Ende des Zwischenstückes 25 wird von einem Konusansatz 28 gebildet, der in den konischen Durchtrittskanal 18 abdichtend einschiebbar ist, während das rückwärtige Ende ein durch Nuten unterbrochenes zylindrisches Kupplungsteil 29 aufweist, auf das die zylindrische Halterung 24 des Schutzbeutels 23 abdichtend aufgeschoben wird.

Im Innern des Schutzbeutels 23 befindet sich gemäss Fig. 2 der am rückwärtigen Ende des Katheters 22 vorgesehene verdickte Katheteransatz 30 zur Ankupplung einer Schlauchleitung. Ein unmittelbar vor dem Katheteransatz 30 angeordneter Zylinder 31 ist nach vorne durch einen Wulst 32 begrenzt, dessen Vorderseite als Schrägschulter, und dessen Rückseite als steile Ringschulter 33 ausgebildet ist. Der Durchmesser des Wulstes 32 ist so bemessen, dass das Muffenteil 14 mit dem Einführungskanal 18 in rückwärtiger Richtung ohne Mühe über den Wulst 32 geschoben werden kann. Wenn das Muffenteil 14 jedoch über den Wulst 32 geschoben worden ist, stösst die Stirnwand 34a des Gewinderaumes gegen die steile Ringschulter 33, so dass diese nicht überwunden werden kann.

Nachdem der Katheter 22 gemäss Fig. 2 aus dem Schutzbeutel 23 heraus durch die Hülse 10 hindurch mit seinem vorderen Ende in die Vene eingeführt worden ist, wird die Halterung 24 des Schutzbeutels 23 von dem Zwischenstück 25 abgezogen und der Schutzbeutel entfernt. Anschliessend wird das Zwischenstück 25 mit seinem Konusansatz 28 aus dem konischen Durchtrittskanal 18 herausgezogen und seine beiden Teile werden durch radiales Auseinanderziehen voneinander getrennt. Schliesslich wird das Muffenteil 14 von dem Hülsenansatz 11 abgeschraubt, so dass die Dichtungsscheibe 15 und der Dichtungszylinder 16, die ja halbseitig radial geschlitzt sind, entfernt werden können. Auch die geschlitzte Hülse 10 einschliesslich des Hülsenansatzes 11 kann von dem Katheter 22 abgenommen werden.

Das Muffenteil 14 wird nun gemäss Fig. 3 zum rückwärtigen Katheterende geführt und über den Wulst 32 gestreift. Sie dient nunmehr der Befestigung des rückwärtigen Katheterendes an der Fixierplatte 34. Diese Fixierplatte 24 weist zwei auf die Haut auflegbare Flügel 37 auf, die an die seitlichen Schenkel eines U-förmigen Halters 35 angesetzt sind, welcher in seiner Mulde den Katheter 22 aufnimmt. Am rückwärtigen Ende des Halters 35 befinden sich radial abstehende Stege 36, die in die Gewindegänge des Muffenteiles 14 eingreifen. Durch Drehen des Muffenteiles 14 kann daher der Halter 35 an dem Muffenteil bis zum Anschlag vorbewegt und befestigt werden. Die Stege 36 haben die gleiche Form und Anordnung wie die Kupplungselemente 13 in Fig. 1a.

Nach dem Befestigen des Katheterendes mit Hilfe des Muffenteiles 14 an der Fixierplatte 34 werden die Flügel 37 mit Heftpflaster auf der Haut des Patienten festgeklebt.

## Patentansprüche

1. Katheterisierungsvorrichtung, mit einem Katheter (22), der an seinem rückwärtigen Ende einen Katheteransatz (30) aufweist, mit einer an ihrem rückwärtigen Ende mit einer eine Kupplungsvorrichtung (13) aufweisenden Hülsenansatz (11) versehen Hülse (10) zum Einführen des Katheters (22), mit einer durch die Hülse (10) hindurchsteckbaren Kanüle (19), die an ihrem vorderen Ende mit einer Schneidkante (20) und an ihrem rückwärtigen Ende mit einem Handgriff (21) versehen ist, und mit einem von dem Katheter (22) zu durchdringenden, bis zum Rand geschlitzten und seitlich von dem Katheter (22) abnehmbaren Dichtungselement, dadurch gekennzeichnet, dass das Dichtungselement eine vor dem Einführen des Katheters (22) von der Kanüle (19) zu durchdringende Dichtungsscheibe (15) ist, die an der rückwärtigen Stirnfläche des Hülsenansatzes (11) der in Längsrichtung teilbaren Hülse (10) angeordnet ist und von einem mit der Kupplungsvorrichtung (13) lösbar ineinandergreifenden Muffenteil (14) gegen die Stirnfläche gedrückt wird, welches an seinem rückwärtigen Ende einen Durchtrittskanal (18) aufweist.

2. Katheterisierungsvorrichtung nach Anspruch 1, dadurch gekennzeichnet, dass der Katheter (22) in der Nähe seines rückwärtigen Endes eine schräge Ringschulter (33) aufweist, über die das Muffenteil (14) nach hinten schiebbar, aber nicht nach vorne zurückbewegbar ist, und dass eine am Körper zu befestigende Fixierplatte (34) vorgesehen ist, die eine mit dem Muffenteil (14) ineinandergreifende zweite Kupplungsvorrichtung (36) aufweist.

3. Katheterisierungsvorrichtung nach Anspruch 1 oder 2, dadurch gekennzeichnet, dass die Hülse (10) einen vor der Dichtungsscheibe (15) angeordneten radial geschlitzten Dichtungszylinder (16) enthält.

4. Katheterisierungsvorrichtung nach einem der Ansprüche 1 bis 3, dadurch gekennzeichnet, dass der Durchtrittskanal (18) des Muffenteiles (14) konisch ausgebildet ist, und dass ein von dem Katheter (22) passierbares teilbares Zwischenstück (25), das an seinem rückwärtigen Ende mit der Halterung (24) eines Schutzbeutels (23) verbunden ist, mit einem nach vorne weisenden hohlen Konusansatz (28) in den Durchtrittskanal (18) eingeschoben ist.

## Claims

1. Catheter assembly comprising a catheter (22) having at its rear end a catheter attachment (30), a sleeve (10) having at its rear end a sleeve projection (11) provided with a coupling means (13) for the introduction of the catheter (22), a cannula (19) insertable through the sleeve (10) and provided at its front end with a cutting edge (20) and at its rear end with a handle (21), and a sealing element to be penetrated by the catheter (22), being slotted to its edge and being removable laterally from the catheter (22), characterized in that the sealing element is a sealing plate (15) to be penetrated by the cannula (19) prior to the introduction of the catheter and being mounted at the rear end face of the sleeve projection (11) of the sleeve (10) dividable longitudinally, said sealing plate being pressed against the end face by a socket element (14) detachably engaging the coupling means (13) and having a channel passage (18) at its rear end.

2. Catheter assembly according to claim 1, characterized in that the catheter (22) comprises nears its rear end an inclined annular shoulder (33) on which the socket element (14) may be slipped to the rear while it cannot be returned to the front, and that a fixing plate (34) to be secured to the body is provided which contains a second coupling means (36) engaging the socket element (14).

3 Catheter assembly according to claim 1 or 2, characterized in that the sleeve (10) contains a radially slotted sealing cylinder (16) arranged in advance of the sealing plate (15).

4. Catheter assembly according to one of claims 1 to 3, characterized in that the channel passage (18) of the socket element (14) is of a conical shape and that a divisable intermediate member (25) traversable by the catheter (22) and being connected at its rear end to the mounting (24) of a protective bag (23) is inserted into the channel passage (18) with a hollow cone attachment (28) pointing to the front.

**Revendications**

1. Dispositif de cathétérisme avec un cathéter (22) comportant à son extrémité arrière un embout de cathéter (30), avec une gaine (10) munie à son extrémité arrière d'un embout (11) doté d'un dispositif d'accouplement (13) pour l'introduction du cathéter (22), avec une canule (19) enfichable dans la gaine (10) et présentant un bord tranchant (20) à son extrémité antérieure et une poignée (21) à son extrémité arrière et avec un élément d'étanchéité que doit traverser le cathéter (22), fendu jusqu'au bord et pouvant être séparé latéralement du cathéter (22), caractérisé en ce que l'élément d'étanchéité est une rondelle d'étanchéité (15) que doit traverser la canule (19) avant l'introduction du cathéter (22), laquelle rondelle est disposée sur la face postérieure de l'embout (11) de la gaine (10) séparable dans le sens de la longueur et pressée contre la face par un élément de manchon (14) qui s'engage de façon démontable dans le dispositif d'accouplement (13), ledit élément de manchon comportant un conduit de passage (18) à son extrémité arrière.

2. Dispositif de cathétérisme selon la revendication 1, caractérisé en ce que le cathéter (22) présente au voisinage de son extrémité arrière un épaulement annulaire oblique (33) par dessus lequel l'élément de manchon (14) peut coulisser vers l'arrière mais sans pouvoir revenir vers l'avant et en ce qu'il est prévu une plaque de fixation (34) destinée à être fixée sur le corps qui comporte un deuxième dispositif d'accouplement (36) qui s'engage dans l'élément de manchon (14).

3. Dispositif de cathétérisme selon la revendication 1 ou 2, caractérisé en ce que la gaine (10) renferme un cylindre d'étanchéité (16) fendu dans le sens radial, disposé devant la rondelle d'étanchéité (15).

4. Dispositif de cathétérisme selon l'une des revendications 1 à 3, caractérisé en ce que le conduit de passage (18) de l'élément de manchon (14) possède une forme conique et en ce qu'une pièce intermédiaire (25) séparable, traversable par le cathéter (22), qui est reliée par son extrémité arrière au support (24) d'un sac de protection (23) est insérée dans le conduit de passage (18) avec un embout conique creux (28) pointé vers l'avant.

0 054 728

FIG. 1

FIG. 1a

FIG. 2

FIG. 3

7